# EUROPEAN PATENT SPECIFICATION

(11) **EP 4 168 073 B1**
(45) Date of publication and mention of the grant of the patent: **04.09.2024**
(21) Application number: 21733472.1
(22) Date of filing: 22.06.2021
(51) Int. Cl.: A61M 5/32

(54) **A SAFETY DEVICE FOR A NEEDLE OF A MEDICAL DEVICE**
SICHERHEITSVORRICHTUNG FÜR EINE NADEL EINER MEDIZINISCHEN VORRICHTUNG
DISPOSITIF DE SÉCURITÉ POUR UNE AIGUILLE D'UN DISPOSITIF MÉDICAL

(30) Priority: 23.06.2020 EP 20315315
(43) Date of publication of application: 26.04.2023
(73) Proprietor: Becton Dickinson France, 38800 Le Pont de Claix (FR)
(72) Inventor: CARREL, Franck, 38220 Saint Jean de Vaulx (FR)
(74) Representative: Novagraaf International SA
(86) International application number: PCT/EP2021/067040
(87) International publication number: WO 2021/259952

(56) References cited:
- EP-A1- 1 433 419
- WO-A1-2016/198387
- US-A1- 2003 078 548

## Description

### FIELD OF THE INVENTION

The present invention relates to a safety device for a needle of a medical device, a safety needle hub and a medical device comprising such a safety device.

### BACKGROUND OF THE INVENTION

A wide number of medical devices rely on a needle in order to prick a patient's skin and to deliver a medicine or to collect a body fluid. In particular, syringes are a common way of delivering drugs or vaccines to patients and usually comprise an injection needle to deliver such drugs into a patient's skin or a patient's muscle. However, needles present a sharp tip with a risk of needle stick injury, in particular for the medical staff, and it is highly desirable to prevent such needle stick injuries for safety reasons.

Consequently, safety devices have been proposed in order to cover the needle when the needle is not to be used and to allow access to the needle when a medical operation is performed. For example, document EP3302656 discloses such a safety device with a pivoting shield that pivot to an open position when a protective cap is removed from the needle and covers the needle in a closed position thanks to a simple push on the shield, thus allowing a safe handling and disposable of the syringe after use.

However, the shield of such a prior art safety device is not locked in the initial position and the protective cap is only maintained by friction on the needle. There is thus a risk of unexpected opening of the safety device before use. In addition, such a prior art safety device relies on a hook to be locked to the needle in a final position. This hook must be accommodated in the protective cap in order to keep the safety device small, but this may decrease the protection and sterility of the needle before use. In addition, EP 1 433 419 A1 relates to a safety needle assembly with pivoting shield.

There is thus a need for a safety device overcoming these drawbacks. In other words, there is a need for a safety device preventing any unexpected opening while maintaining a high level of protection and sterility of the needle before use. In addition, such a safety device must remain small, to limit storage space as well as convenient and safe to use, in particular with gloves and during emergency situations.

### SUMMARY OF THE INVENTION

This objective is accomplished by a safety device for a needle of a medical device, the safety device comprising:
- a ring arranged to be fixed with regard to the needle
- a shield arranged to be mounted on the ring by a pivot link so as to define a closed position in which the shield covers the needle and an open position in which the shield does not cover the needle
- a protective cap configured to surround the needle and to accommodate at least part of the shield in the closed position

wherein the ring and the shield further comprise a locking unit adapted to lock the shield to the ring in the closed position, and
wherein the protective cap and the shield comprise a releasing unit adapted to release the locking unit during a portion of a removal movement of the protective cap.

Thanks to the locking unit, the protective cap cannot be moved to the open position unintentionally in the initial configuration of the safety device wherein the protective cap is present and the shield is in the closed position. Further, when the safety device is in a final configuration after use without the protective cap and with the shield in the closed position, the locking unit can lock the shield to the ring and no hook is thus required on the shield. Finally, the releasing unit releases the locking unit during the removal movement of the protective cap from the needle and/or from the safety device, which renders the present safety device efficient and straightforward to use.

Advantageously, the locking unit comprises:
at least one recess provided on one of the ring and the shield
at least one peg provided on the other of the ring and the shield
wherein in the closed position, the peg is engaged into the recess.

This locking unit is simple to manufacture and provides a safe locking of the shield to the ring. Preferably, two pegs and two recesses are provided, for example on opposite sides of the shield and the ring, respectively. For example, the locking unit consists in one or two recesses provided on the ring and one or two pegs provided on the shield.

Advantageously, the releasing unit comprises a distal protrusion provided on the protective cap and defining a deflecting surface, and a flexible wing provided on the shield and comprising the peg, the deflecting surface being configured to deflect the flexible wing during the portion of the removal movement of the protective cap so as to disengage the peg from the recess.

These releasing unit allows for a straightforward and intuitive release of the locking member during the portion of the removal movement of the protective cap.

Preferably, the deflection of the flexible wing is performed in the transversal or outward direction. The flexible wing may comprise a sliding surface adapted for the deflecting surface to slide on it. For example, both the sliding surface and the deflecting surface may have the same slope. Such a sliding surface allows to decrease the force required to remove the protective cap from the safety device.

Advantageously, the protective cap and the shield comprise an opening unit adapted to move the shield from the closed position to the open position during a subsequent portion of the removal movement of the protective cap. This opening unit can thus perform a passive opening of the shield, without a direct operation of the shield by the user, which significantly increases the safety and the convenience of operating such a safety device.

Advantageously, the opening unit comprises a cam surface provided on the shield and a pusher provided on the protective cap, the pusher being configured to engage the cam surface during the subsequent portion of the removal movement of the protective cap. Preferably, the cam surface is located on the shield remotely from the pivot link so as to produce a lever effect.

This pushing unit is simple to manufacture and allows a reliable passive opening of the shield in the subsequent portion of the removal movement of the protective cap. Preferably, the cam surface is provided on the flexible wing, such as on a proximal edge of the flexible wing and for example proximally from the sliding surface and/or from the peg.

Advantageously, the safety device comprises a safety unit adapted to ease the engagement the locking unit when the shield is moved from the open position to the closed position. Consequently, the shield can be blocked permanently in the closed position covering the needle in the final configuration thanks to a limited force applied on the shield, therefore allowing a convenient handling and a safe disposal of the safety device.

Advantageously, the safety unit comprises the flexible wing of the shield and a distal sloped surface provided on the ring adapted to deflect the flexible wing when the shield is moved from the open position to the closed position. For example, the peg can engage the recess once again with a limited force. Preferably, the safety unit also comprises the sliding surface of the flexible wing. Such a safety unit is simple to manufacture and only requires a limited force for re-engaging the locking unit in the final configuration.

Advantageously, the protective cap and the shield further comprise an assembly unit adapted to fasten or attach the shield to the protective cap, when the shield is in the closed position and/or the safety device is in the initial configuration. Such an assembly unit contributes to prevent any movement of the shield as long as the protective cap is not removed and also allows to assemble the safety device before the safety device is mounted on a medical device.

Advantageously, the assembly unit comprises a distal tong provided on one of the shield and the protective cap and a longitudinal opening provided on the other of the shield and the protective cap, wherein the distal tong is accommodated into the longitudinal opening before the protective cap is removed from the shield, i.e. in the initial configuration of the shield. This assembly unit is easy to manufacture and to assemble.

Advantageously, the safety device further comprises a guiding unit configured to provide a sliding engagement between the ring and the protective cap. Such a guiding unit can prevent any undesired movement of the protective cap during the removal movement of the shield and for example establishes a prismatic joint between the ring and the protective cap. Preferably, the guiding unit comprises a longitudinal slot on one of the ring and the protective cap and a longitudinal rib on the other of the ring and the protective cap, preferably on the protective cap.

Advantageously, the ring has an edge or circumference and the pivot link is provided on a portion of the circumference, which allows for an easy assembly of the shield to the ring and a reliable rotation of the shield.

Advantageously, the locking unit comprise two recesses provided on two opposite portions of the circumference of the ring, which provide a safe engagement of the locking unit. Consequently, the shield may also comprise two opposite flexible wings each provided with a peg.

Preferably, the recesses are provided on a different portion of the circumference of the ring than the pivot link. For example, the ring comprises opposite side surfaces and the recesses are provided on theses side surfaces.

Advantageously, the protective cap comprises a needle cap adapted to receive the needle, and the protective cap comprises a cap opening adapted to receive at least part of the shield in the closed position. This allows to reduce the volume of the safety device and thus not to change storage and transportation practice. Preferably, the needle cap has a single proximal opening adapter to receive the needle and for example, part of a distal neck of the medical device. Consequently, the needle cap may not comprise any opening to receive the shield or part of the shield.

Preferably, the protective cap is adapted to fully accommodate the shield. For example, the protective cap defines a diameter and the shield is comprised within said diameter in the initial configuration. For example, the diameter is a diameter of the ring and is equal or less than a diameter such as an external diameter of the medical device on which the present safety device is intended to be mounted.

A second aspect of the present invention is a safety needle hub adapted to be fixed on a medical device such as a syringe, the safety needle hub comprising a needle and a safety device according to the first aspect of the present invention.

A third aspect of the present invention is a medical device adapted to inject and/or remove a fluid from a body, comprising a needle and a safety device according to the first aspect of the present invention or a safety needle hub according to the second aspect of the present invention.

Advantageously, the shield comprises a longitudinal axis parallel to the needle in the closed position, which provides a compact and user-friendly safety needle hub or medical device.

### BRIEF DESCRIPTION OF THE DRAWINGS

Further advantages and preferred embodiments of the present invention will become apparent from the following detailed description and drawings, in which:
Figure 1 is a perspective view of the safety device according to the present invention assembled on a syringe as a medical device.
Figure 2 is a detailed perspective view of a ring of the safety device of Figure 1.
Figure 3 is a detailed perspective view of a shield of the safety device of Figure 1.
Figure 4 is a detailed perspective view of a protective cap of the safety device of Figure 1.
Figure 5 is a detailed side view of the safety device of Figure 1, in an initial configuration.
Figure 6 is a top cross-sectional view of the safety device of Figure 5 along a A-A' cross-section.
Figure 7 is an enlarged partial view of Figure 6.
Figure 8 is a detailed view of the safety device of Figure 1 after the portion of a removal movement of the protective cap.
Figure 9 is a top cross-sectional view of the safety device of Figure 8 along a A-A' cross-section.
Figure 10 is an enlarged partial view of Figure 9.
Figure 11 is a detailed view of the safety device of Figure 1 in the subsequent portion of a removal movement of the protective cap.
Figure 12 is a detailed view of the safety device of Figure 1 after removal of the protective cap, with the shield is in the open position.
Figure 13 is a detailed view of the safety device of Figure 1 in a final configuration.

### DETAILED DESCRIPTION

The present safety device is intended to be used with or on any kind of injection, test or sampling medical device using a needle adapted to prick a patient's body for any kind of prophylactic, diagnosis, aesthetics or therapeutic medical treatment. For example, such a medical device can be a medical syringe or a blood collection tube. The safety device can be delivered mounted on the medical device or as a safety needle hub adapted to be fixed on a tip of the medical device. In addition, the safety device can also be proposed alone, for example for a subsequent mounting on a syringe or on a medical device, depending on the targeted customer.

The safety device according to the present invention is described in the examples of the appended figures as mounted on a syringe as a medical device. As such, in this application, the distal direction must be understood as the direction of injection with reference to the medical device, and the proximal direction is the opposite direction, i.e. the direction toward the hand of the medical caregiver or of the patient.

### Description of the Main Embodiment

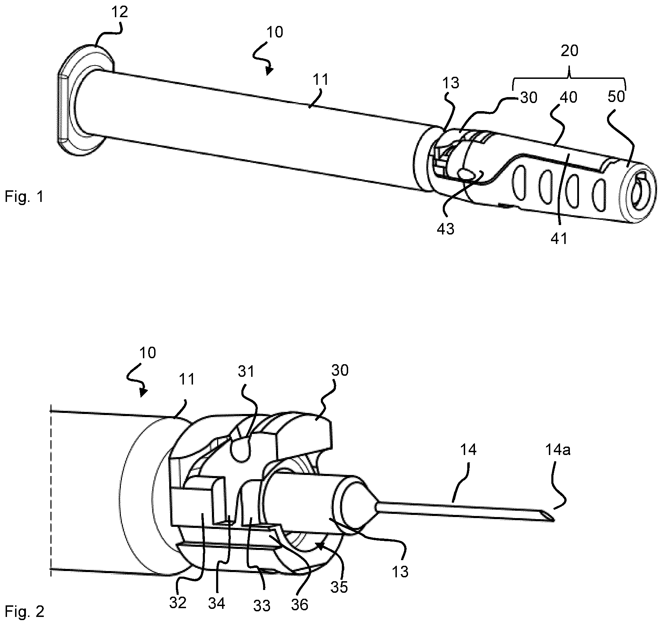

Now referring to Fig. 1, and from proximal to distal or from left to right in the view of Fig. 1, a medical device under the form of a syringe 10 comprises a proximal flange 12, a barrel 11, a distal neck 13 and an embodiment of a safety device 20 comprising a ring 30, a shield 40 and a protective cap 50.

With reference to Fig. 2, the ring 30 is mounted around the distal neck 13, from which protrudes a needle 14 comprising a tip 14a. The ring 30 can comprise a knuckle 31 adapted to accommodate part the shield 40 in order to form a pivot link such as a hinge. Further, the ring 30 can comprise two side surfaces 32 located on two opposite sides of the ring 30 (only one visible in Fig. 2). The side surfaces may each have a distal sloped surface 33 and a recess 34 located proximally to the distal sloped surface 33.

Each of the recesses 34 can have a sloped distal side proximally from the distal sloped surface 33 (on the right of the recess in the figures), for example pointing the distal direction, and a straight proximal side (on the left of the recess in the figures). The ring 30 is preferably made of hard, rigid material such as a hard polymer or composite adapted for medical use, such as high density polyethylene (PE), polypropylene (PP), polycarbonate (PC), acrylonitrile butadiene styrene (ABS), polyoxymethylene (POM), polystyrene (PS), polybutylene terephthalate (PBT), polyamide (PA), and their combinations.

The ring 30 may include a central opening 35 located around the distal neck 13 and opened distally. For example, the central opening 35 can have a radial opening toward the side surfaces 32 and the distal sloped surfaces 33. Finally, the ring 30 may further comprise two longitudinal slots 36, located on opposite sides of the ring 30, for example nearby or below the side surfaces 32.

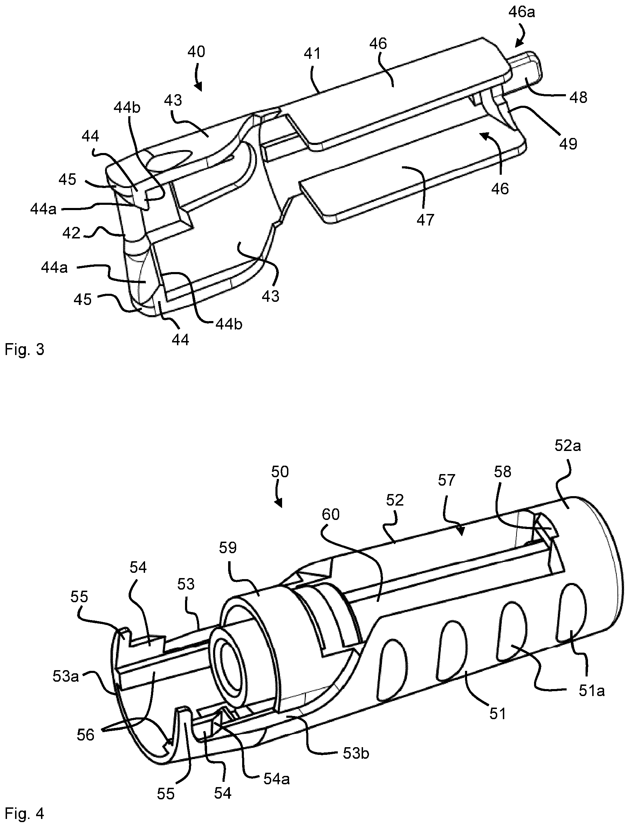

With reference to Fig. 3, the shield 40 comprises a main surface 41 (the top surface of the shield 40 in Fig. 1, the back surface of the shield 40 in Fig. 3), a pivot 42 located at a proximal extremity of the main surface 41 and adapted to be received in the knuckle 31 of the ring 30 so as to form the pivot link. The shield 40 further includes on its proximal portion two flexible wings 43 extending from the main surface 41, preferably located each on an opposite side of the pivot 42. The flexible wings 43 may be integral with the shield 40 and can comprise a type of material and a thickness allowing a deformation, in particular in the transversal direction.

The flexible wings 43 each define at their proximal extremity a peg 44 defining a sliding surface 44a and a straight, transversal distal surface 44b intended to act as a stop. Each flexible wing 43 further defines a cam surface 45 extending proximally with regard to the pegs 44.

In addition, the distal portion of the shield 40 (on the right in Fig. 3) further comprises a shield recess 46 defined by two side walls 47 extending from the main surface 41. The shield recess 46 has a substantially open distal end 46a which is partially closed by a transversal wall 49. The shield 40 may also comprise a distal tong 48 extending from the distal extremity of the main surface 41, further from the transversal wall 49. The shield 40 can be made of the same material as the ring 30 and be preferably less rigid than the ring 30, for example through the material thickness and/or composition.

With reference to Fig. 4, the protective cap 50 comprises a main surface 51 which is substantially U-shaped in the distal portion 52 of the protective cap 50 (on the right in Fig. 4) and hemi circular in the proximal portion 53 (on the left in Fig. 4). The proximal extremity 53a of the proximal portion 53 is provided with two pushers 55 and two distal protrusions 54.

The distal protrusions 54 are extending distally from the pusher 55 and each cover part of an edge 53b of the proximal portion 53. The distal protrusions 54 each define a sloped or deflecting surface 54a facing an outside of the protective cap 50. For example, the deflecting surface 54a has the same angle as the sliding surface 44a of the shield 40. The pushers 55 extend circumferentially from the edge 53b of the proximal portion 53 and may have a curved shape similar or complementary to the shape of the cam surface 45 of the shield 40.

The proximal portion further comprises two longitudinal ribs 56 extending on the opposite sides of the proximal portion 53 and on the inside of the main surface 51, for example below and parallel to the distal protrusions 54. The distal portion 52 of the protective cap 50 comprises a cap opening 57, at the top of the "U" shape and a longitudinal opening 58 optionally provided at the circular distal extremity 52a of the distal portion 52. Notches 51a and/or protrusions can be provided on the sides of the main surface 51, to facilitate grasping. The protective cap 50 can comprise the same material as the ring 30.

A needle cap 60 may be accommodated inside the protective cap 50 and is maintained for example by friction with the internal side of the main surface 51 and with an arch 59 of the protective cap 50. The needle cap 60 is thus intended to be fixed with regard to the protective cap 50 and may also be glued to the protective cap 50 or the protective cap 50 can be overmolded on the needle cap 60. The needle cap 60 is intended to accommodate the needle 14 during the storage time of the syringe 10 and thus only comprises a single proximal opening 61 and a proximal edge 62 intended to contact the distal neck 13 in the initial configuration. The needle cap 60 can comprise elastomeric polymer or elastomer such as natural rubber, butyl rubber or silicon rubber.

When the safety device 20 is assembled on the syringe 10, as shown in Fig 1 and Figs. 5, 6 and 7, the needle 14 is accommodated into the needle cap 60 which is inserted in the central opening 35 of the ring 30 and contacts the distal neck 13 of the syringe 10 in order to maintain the needle 14 protected from dust and microorganisms. The needle cap 60 is accommodated in the protective cap 50, which partially surrounds the ring 30 and the longitudinal ribs 56 of the protective cap 50 are engaged into the longitudinal slots 36 of the ring 30.

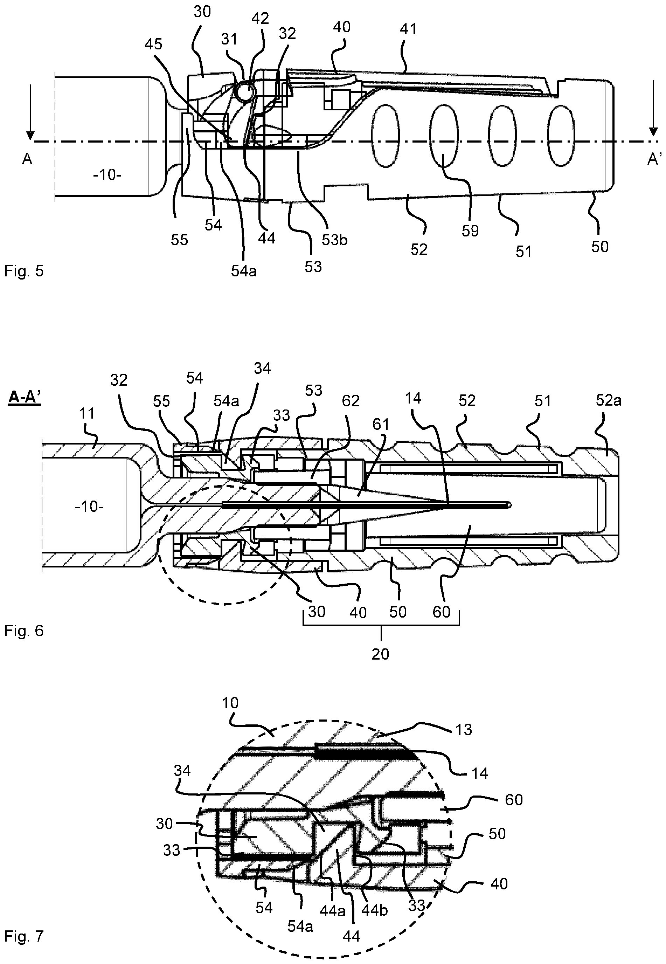

The shield 40 may be partially accommodated into the protective cap 50: the flexible wings 43 of the shield 40 may contact the edge 53b of the protective cap 50, the two side walls 47 of the shield 40 are accommodated into the cap opening 57 of the protective cap 50 and the needle cap 60 is partially accommodated in the shield recess 46 of the shield 40. The distal tong 48 of the shield 40 is accommodated into the longitudinal opening 58 of the protective cap 50 (not visible in Figs. 5-7) which allows to maintain the shield 40 fixed with regard to the protective cap 50 even when the safety device 20 is not assembled to a medical device. The distal tong 48 and the longitudinal opening 58 may thus act as an assembly unit.

Further, the shield 40 is assembled to the ring 30 by the pivot 42, which is in a pivot engagement with the knuckle 31 and locked to the ring 30 by the engagement of the pegs 44 into the recesses 34: in this closed position visible in Figs. 1 and 5 to 7, any rotation of the shield 40 with regard to the ring 30 is prevented. In particular, the distal surface 44b of the pegs 44 can contact or abut the sloped distal side of the recess 34 and the shield 40 cannot rotate toward the open position. The pegs 44 and the side surfaces 32 thus act as a locking unit.

### Operation of the Embodiment

During storage time, the syringe can be provided into a blister with the safety device in the initial configuration of Fig. 1 and 5 to 7: the shield 40 is in the closed position, locked to the ring 30 and the protective cap 50 is fixed to the shield 40 and accommodates the needle 14. At the time of being used, a user must first remove the protective cap 50 from the distal neck 13 in order to reveal the needle 14, as usual. To that end, the user can grip the main surface 51 of the cap with his/her fingers, for example on the notches 51a, and move the protective cap 50 in the distal direction (on the right of the figures).

Thanks to the engagement between the longitudinal slots 36 of the ring 30 and the longitudinal ribs 56 of the protective cap 50, only a linear movement in the distal direction of the protective cap is possible and the longitudinal slots 36 and the longitudinal ribs 56 thus acts as a guiding unit establishing a sliding engagement or prismatic joint between the ring 30 and the protective cap 50 and the removal movement of the protective cap 50 is thus limited to a linear movement in the distal direction.

During the removal movement of the protective cap 50 from the ring 30 and the needle 14, a portion of this movement results in the disengagement or release of the locking unit and a subsequent portion of this movement results in the rotation of the shield from the closed position to the open position.

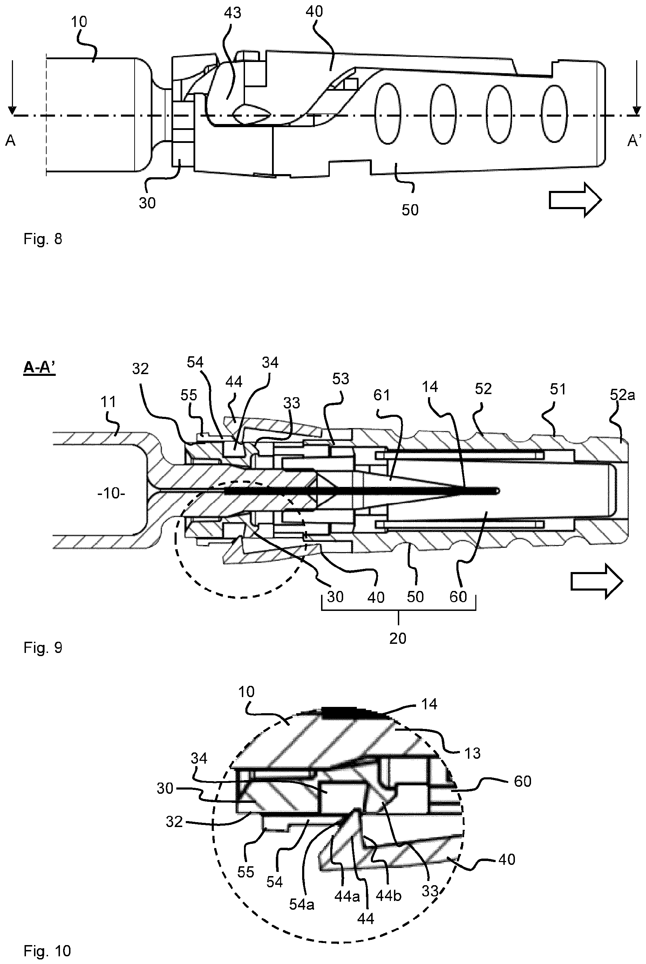

With reference to Figs. 8 to 10, the protective cap 50 is moved distally (see the white arrows in Figs. 8 and 9) and the shield 40 remains in the closed position of Figs. 5-7, in particular thanks to the engagement of the pegs 44 of the shield 40 into the recesses 34 of the ring 30 and the preferential engagement of the distal tong 48 into the longitudinal opening 58.

In this portion of the removal movement of the protective cap 50, the distal protrusions 54 move distally and come in contact with the pegs 44. As the removal movement of the protective cap 50 is maintained, the deflecting surfaces 54a of the distal protrusions 54 slide against the respective sliding surface 44a of the pegs 44 and deflect outwardly the flexible wings 43 of the shield. Consequently, the flexible wings 43 and optionally the sliding surface 44a form with the distal protrusion 54 and the deflecting surface 54a a releasing unit configured to release or disengage the locking unit when the protective cap 50 is removed from the needle 14.

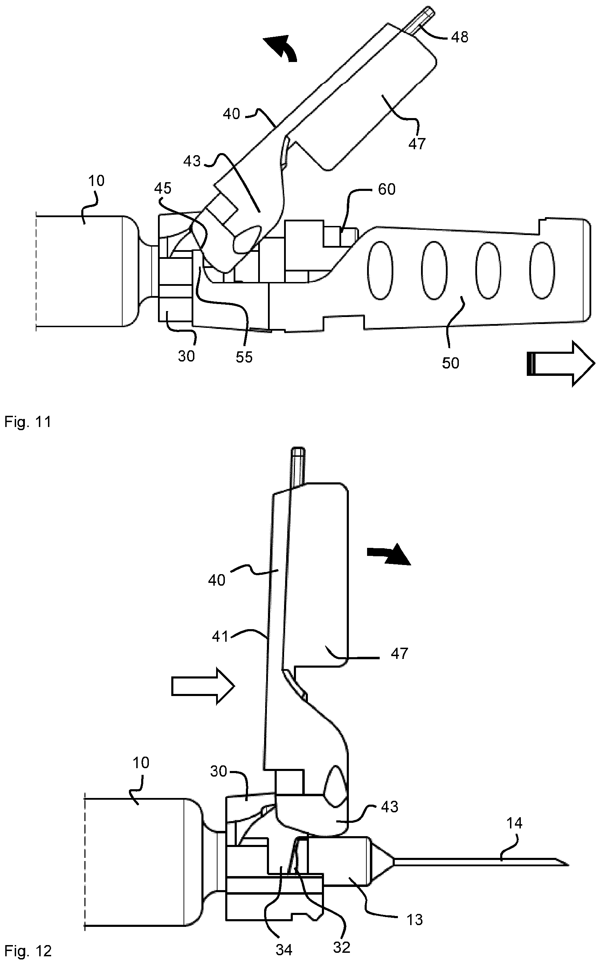

After the release of the locking unit, the shield 40 is free to rotate with regard to the ring 30: during the subsequent portion of the removal movement of the protective cap 50, the pushers 55 come in contact with the cam surfaces 45 of the shield 40 (see Fig. 11). The cam surfaces 45 are pushed by the pushers 55, which rotates the shield 40 toward the proximal direction (see the black arrow in Fig. 11) from the closed position to an open position visible in Fig. 12.

At the end of the subsequent portion of the removal movement of the protective cap 50, the shield may have rotated proximally for example of at least 90° and preferably 120 or 140° with regard to its closed position: the safety device 20 is now in an operating configuration, the needle 14 is now accessible and can be used to prick the patient's body and a medicine can be injected thanks to the syringe 10.

After the injection operation has been performed, the shield 40 can be rotated back toward the needle 14. For example, the user can press distally the main surface 41 of the shield 40 (see the white arrow in Fig. 12) and the shield 40 can rotate toward the distal direction (see the black arrow in Fig 12).

The locking unit can then be locked again thanks by an addition force applied on the main surface 41 of the shield 40 and the flexible wings 43 may be deflected thanks to the contact between the sliding surfaces 44a of the pegs 44 and the distal sloped surfaces 33 of the ring 30. The pegs 44 finally engage the recesses 34 similarly to the initial position of Figs. 5 to 7 and the shield 40 is then in a closed position, covering the needle 14 and preventing any needle stick injury.

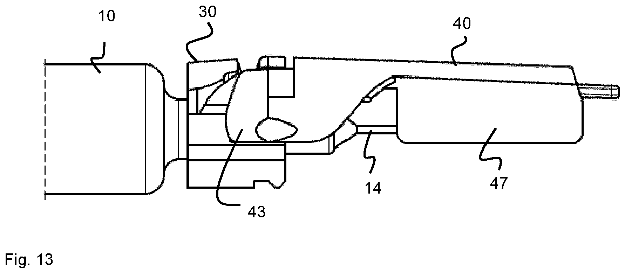

In the final configuration of Fig. 13, the shield 40 is in the same closed position as in the initial configuration of Figs. 1 and 5-7. Consequently, no direct engagement between the shield and the needle is required and the locking unit can be used both in the initial configuration and in the final configuration of the safety device 20. This allows the safety device to prevent needle stick injuries in the final configuration, to be compact at the time of storage and disposal and to efficiently protect the needle in the initial configuration.

Although the present invention has been described and illustrated in detail, it is clearly understood that the same is by way of illustration and example only and is not to be taken by way of limitations, the scope of the present invention being limited only by the terms of the appended claims. For example, the elements of the safety device such as the pegs, the recesses, the pushers, etc. may have any shape or geometry as long as their function can be performed.

## Claims

1. A safety device (20) for a needle (14) of a medical device, the safety device (20) comprising:
• a ring (30) arranged to be fixed with regard to the needle (14)
• a shield (40) arranged to be mounted on the ring (30) by a pivot link so as to define a closed position in which the shield (40) covers the needle (14) and an open position in which the shield (40) does not cover the needle (14)
• a protective cap (50) configured to surround the needle (14) and to accommodate at least part of the shield (40) in the closed position
**characterized in that** the ring (30) and the shield (40) further comprise a locking unit adapted to lock the shield (40) to the ring (30) in the closed position, and
**in that** the protective cap (50) and the shield (40) comprise a releasing unit adapted to release the locking unit during a portion of a removal movement of the protective cap (50).

2. The safety device (20) according to the previous claim, wherein the locking unit comprise:
• at least one recess (34) provided on one of the ring (30) and the shield (40)
• at least one peg (44) provided on the other of the ring (30) and the shield (40)
wherein in the closed position, the peg (44) is engaged into the recess (34).

3. The safety device (20) according to the previous claim, wherein the releasing unit comprises a distal protrusion (54) provided on the protective cap (50) and defining a deflecting surface (54a), and a flexible wing (43) provided on the shield (40) and comprising the peg (44), the deflecting surface (54a) being configured to deflect the flexible wing (43) during the portion of the removal movement of the protective cap (50) so as to disengage the peg (44) from the recess (34).

4. The safety device (20) according to any of the previous claims, wherein the protective cap (50) and the shield (40) comprise an opening unit adapted to move the shield (40) from the closed position to the open position during a subsequent portion of the removal movement of the protective cap (50).

5. The safety device (20) according to the previous claim, wherein the opening unit comprises a cam surface (45) provided on the shield (40) and a pusher (55) provided on the protective cap (50), the pusher (55) being configured to engage the cam surface (45) during the subsequent portion of the removal movement of the protective cap (50).

6. The safety device (20) according to any of the previous claims, wherein the safety device (20) comprises a safety unit adapted to ease the engagement of the locking unit when the shield (40) is moved from the open position to the closed position.

7. The safety device (20) according to the previous claim and claims 3 to 5, wherein the safety unit comprise the flexible wing (43) of the shield (40) and a distal sloped surface (33) provided on the ring (30) and adapted to deflect the flexible wing (43) when the shield (40) is moved from the open position to the closed position.

8. The safety device (20) according to any of the previous claims, wherein the protective cap (50) and the shield (40) further comprise an assembly unit adapted to attach the shield (40) to the protective cap (50), when the shield (40) is in the closed position.

9. The safety device (20) according to the previous claim, wherein the assembly unit comprises a distal tong (48) provided on one of the shield (40) and the protective cap (50) and a longitudinal opening (58) provided on the other of the shield (40) and the protective cap (50), wherein the distal tong (48) is accommodated into the longitudinal opening (58) before the protective cap (50) is removed from the shield (40).

10. The safety device (20) according to any of the previous claims, wherein the safety device (20) further comprises a guiding unit configured to provide a sliding engagement between the ring (30) and the protective cap (50).

11. The safety device (20) according to any of the previous claims, wherein the ring (30) has a circumference and wherein the pivot link is provided on a portion of the circumference.

12. The safety device (20) according to the previous claim and claims 2 to 10, wherein the locking unit comprises two recesses (34) provided on two opposite portions of the circumference of the ring (30).

13. The safety device (20) according to any of the previous claims, wherein the protective cap (50) comprises a needle cap (60) adapted to receive the needle (14), and wherein the protective cap (50) comprises a cap opening (57) adapted to receive at least part of the shield (40) in the closed position.

14. A safety needle hub adapted to be fixed on a medical device, the safety needle hub comprising a needle (14) and a safety device (20) according to any one of the previous claims.

15. A medical device adapted to inject and/or remove a fluid from a body, the medical device comprising a needle (14) and a safety device (20) according to any one of claims 1 to 13.

16. The safety needle hub of claim 14 or the medical device of claim 15, wherein the shield (40) comprises a longitudinal axis parallel to the needle (14) in the closed position.

## Patentansprüche

1. Sicherheitsvorrichtung (20) für eine Nadel (14) einer medizinischen Vorrichtung, wobei die Sicherheitsvorrichtung (20) Folgendes umfasst:
• einen Ring (30), der angeordnet ist, mit Bezug auf die Nadel (14) fixiert zu sein
• eine Abschirmung (40), die angeordnet ist, mit einem Schwenkglied am Ring (30) montiert zu sein, um eine geschlossene Position, in der die Abschirmung (40) die Nadel (14) abdeckt, und eine geöffnete Position, in der die Abschirmung (40) die Nadel (14) nicht abdeckt, zu definieren
• eine Schutzkappe (50), die dazu ausgelegt ist, die Nadel (14) zu umgeben und mindestens einen Teil der Abschirmung (40) in der geschlossenen Position aufzunehmen
**dadurch gekennzeichnet, dass** der Ring (30) und die Abschirmung (40) ferner eine Verriegelungseinheit umfassen, die angepasst ist, die Abschirmung (40) in der geschlossenen Position am Ring (30) zu verriegeln, und
dadurch, dass die Schutzkappe (50) und die Abschirmung (40) eine Freigabeeinheit umfassen, die angepasst ist, die Verriegelungseinheit während eines Abschnitts einer Entfernungsbewegung der Schutzkappe (50) freizugeben.

2. Sicherheitsvorrichtung (20) nach dem vorhergehenden Anspruch, wobei die Verriegelungseinheit Folgendes umfasst:
• mindestens eine Vertiefung (34), die an einem des Rings (30) und der Abschirmung (40) bereitgestellt ist
• mindestens einen Zapfen (44), der am anderen des Rings (30) und der Abschirmung (40) bereitgestellt ist
wobei der Zapfen (44) in der geschlossenen Position in die Vertiefung (34) eingreift.

3. Sicherheitsvorrichtung (20) nach dem vorhergehenden Anspruch, wobei die Freigabeeinheit einen distalen Vorsprung (54), der auf der Schutzkappe (50) bereitgestellt ist und eine ableitende Fläche (54a) definiert, und einen flexiblen Flügel (43) umfasst, der auf der Abschirmung (40) bereitgestellt ist und den Zapfen (44) umfasst, wobei die ableitende Fläche (54a) dazu ausgelegt ist, den flexiblen Flügel (43) während des Abschnitts der Entfernungsbewegung der Schutzkappe (50) abzuleiten, um den Zapfen (44) aus dem Eingriff in die Vertiefung (34) zu lösen.

4. Sicherheitsvorrichtung (20) nach einem der vorhergehenden Ansprüche, wobei die Schutzkappe (50) und die Abschirmung (40) eine Öffnungseinheit umfassen, die angepasst ist, die Abschirmung (40) während eines folgenden Abschnitts der Entfernungsbewegung der Schutzkappe (50) aus der geschlossenen Position in die geöffnete Position zu bewegen.

5. Sicherheitsvorrichtung (20) nach dem vorhergehenden Anspruch, wobei die Öffnungseinheit eine Nockenfläche (45), die an der Abschirmung (40) bereitgestellt ist, und einen Schieber (55), der an der Schutzkappe (50) bereitgestellt ist, umfasst, wobei der Schieber (55) dazu ausgelegt ist, während des folgenden Abschnitts der Entfernungsbewegung der Schutzkappe (50) in die Nockenfläche (45) einzugreifen.

6. Sicherheitsvorrichtung (20) nach einem der vorhergehenden Ansprüche, wobei die Sicherheitsvorrichtung (20) eine Sicherheitseinheit umfasst, die angepasst ist, das Eingreifen der Verriegelungseinheit zu vereinfachen, wenn die Abschirmung (40) aus der geöffneten Position in die geschlossene Position bewegt wird.

7. Sicherheitsvorrichtung (20) nach dem vorhergehenden Anspruch und den Ansprüchen 3 bis 5, wobei die Sicherheitseinheit den flexiblen Flügel (43) der Abschirmung (40) und eine distale geneigte Fläche (33) umfasst, die am Ring (30) bereitgestellt und angepasst ist, den flexiblen Flügel (43) abzuleiten, wenn die Abschirmung (40) aus der geöffneten Position in die geschlossene Position bewegt wird.

8. Sicherheitsvorrichtung (20) nach einem der vorhergehenden Ansprüche, wobei die Schutzkappe (50) und die Abschirmung (40) ferner eine Montageeinheit umfassen, die angepasst ist, die Abschirmung (40) an der Schutzkappe (50) zu befestigen, wenn sich die Abschirmung (40) in der geschlossenen Position befindet.

9. Sicherheitsvorrichtung (20) nach dem vorhergehenden Anspruch, wobei die Montageeinheit eine distale Zange (48) umfasst, die an einem der Abschirmung (40) und der Schutzkappe (50) bereitgestellt ist, und eine Längsöffnung (58), die am anderen der Abschirmung (40) und der Schutzkappe (50) bereitgestellt ist, umfasst, wobei die distale Zange (48) in die Längsöffnung (58) aufgenommen wird, bevor die Schutzkappe (50) aus der Abschirmung (40) entfernt wird.

10. Sicherheitsvorrichtung (20) nach einem der vorhergehenden Ansprüche, wobei die Sicherheitsvorrichtung (20) ferner eine Führungseinheit umfasst, die dazu ausgelegt ist, einen Gleiteingriff zwischen dem Ring (30) und der Schutzkappe (50) bereitzustellen.

11. Sicherheitsvorrichtung (20) nach einem der vorhergehenden Ansprüche, wobei der Ring (30) einen Umfang aufweist und wobei das Schwenkglied an einem Abschnitt des Umfangs bereitgestellt ist.

12. Sicherheitsvorrichtung (20) nach dem vorhergehenden Anspruch und den Ansprüchen 2 bis 10, wobei die Verriegelungseinheit zwei Vertiefungen (34) umfasst, die auf zwei gegenüberliegenden Abschnitten des Umfangs des Rings (30) bereitgestellt sind.

13. Sicherheitsvorrichtung (20) nach einem der vorhergehenden Ansprüche, wobei die Schutzkappe (50) einen Nadelkappe (60) umfasst, die angepasst ist, die Nadel (14) zu empfangen, und wobei die Schutzkappe (50) eine Kappenöffnung (57) umfasst, die angepasst ist, in der geschlossenen Position mindestens einen Teil der Abschirmung (40) zu empfangen.

14. Sicherheitsnadelnabe, die angepasst ist, an einer medizinischen Vorrichtung fixiert zu sein, wobei die Sicherheitsnadelnabe eine Nadel (14) und eine Sicherheitsvorrichtung (20) nach einem der vorhergehenden Ansprüche umfasst.

15. Medizinische Vorrichtung, die angepasst ist, ein Fluid in einen Körper einzuspritzen und/oder aus demselben zu entfernen, wobei die medizinische Vorrichtung eine Nadel (14) und eine Sicherheitsvorrichtung (20) nach einem der Ansprüche 1 bis 13 umfasst.

16. Sicherheitsnadelnabe nach Anspruch 14 oder medizinische Vorrichtung nach Anspruch 15, wobei die Abschirmung (40) eine Längsachse umfasst, die in der geschlossenen Position parallel zur Nadel (14) verläuft.

## Revendications

1. Dispositif de sécurité (20) pour une aiguille (14) d'un dispositif médical, le dispositif de sécurité (20) comprenant :
une bague (30) agencée pour être fixe par rapport à l'aiguille (14),
une protection (40) agencée pour être montée sur la bague (30) par une liaison de pivot afin de définir une position fermée dans laquelle la protection (40) recouvre l'aiguille (14) et une position ouverte dans laquelle la protection (40) ne recouvre pas l'aiguille (14),
un capuchon de protection (50) configuré pour entourer l'aiguille (14) et pour loger au moins une partie de la protection (40) dans la position fermée,
**caractérisé en ce que** la bague (30) et la protection (40) comprennent en outre une unité de verrouillage adaptée pour verrouiller la protection (40) sur la bague (30) dans la position fermée, et
**en ce que** le capuchon de protection (50) et la protection (40) comprennent une unité de libération adaptée pour libérer l'unité de protection pendant une partie d'un mouvement de retrait du capuchon de protection (50).

2. Dispositif de sécurité (20) selon la revendication précédente, dans lequel l'unité de verrouillage comprend :
au moins un évidement (34) prévu sur l'une parmi la bague (30) et la protection (40),
au moins une cheville (44) prévue sur l'autre parmi la bague (30) et la protection (40),
dans lequel, dans la position fermée, la cheville (44) est mise en prise dans l'évidement (34).

3. Dispositif de sécurité (20) selon la revendication précédente, dans lequel l'unité de libération comprend une saillie distale (54) prévue sur le capuchon de protection (50) et définissant une surface de déviation (54a), et une aile flexible (43) prévue sur la protection (40) et comprenant la cheville (44), la surface de déviation (54a) étant configurée pour dévier l'aile flexible (43) pendant la partie du mouvement de retrait du capuchon de protection (50) afin de dégager la cheville (44) de l'évidement (34).

4. Dispositif de sécurité (20) selon l'une quelconque des revendications précédentes, dans lequel le capuchon de protection (50) et la protection (40) comprennent une unité d'ouverture adaptée pour déplacer la protection (40) de la position fermée à la position ouverte, pendant une partie consécutive du mouvement de retrait du capuchon de protection (50) .

5. Dispositif de sécurité (20) selon la revendication précédente, dans lequel l'unité d'ouverture comprend une surface de came (45) prévue sur la protection (40) et un poussoir (55) prévu sur le capuchon de protection (50), le poussoir (55) étant configuré pour mettre en prise la surface de came (45) pendant la partie consécutive du mouvement de retrait du capuchon de protection (50).

6. Dispositif de sécurité (20) selon l'une quelconque des revendications précédentes, dans lequel le dispositif de sécurité (20) comprend une unité de sécurité adaptée pour faciliter la mise en prise de l'unité de verrouillage, lorsque la protection (40) est déplacée de la position ouverte à la position fermée.

7. Dispositif de sécurité (20) selon la revendication précédente et les revendications 3 à 5, dans lequel l'unité de sécurité comprend l'aile flexible (43) de la protection (40) et une surface inclinée distale (33) prévue sur la bague (30) et adaptée pour dévier l'aile flexible (43) lorsque la protection (40) est déplacée de la position ouverte à la position fermée.

8. Dispositif de sécurité (20) selon l'une quelconque des revendications précédentes, dans lequel le capuchon de protection (50) et la protection (40) comprennent en outre une unité d'assemblage adaptée pour fixer la protection (40) au capuchon de protection (50), lorsque la protection (40) est dans la position fermée.

9. Dispositif de sécurité (20) selon la revendication précédente, dans lequel l'unité d'assemblage comprend une pince distale (48) prévue sur l'un parmi la protection (40) et le capuchon de protection (50) et une ouverture longitudinale (58) prévue sur l'autre parmi la protection (40) et le capuchon de protection (50), dans lequel la pince distale (48) est logée dans l'ouverture longitudinale (58) avant que le capuchon de protection (50) ne soit retiré de la protection (40).

10. Dispositif de sécurité (20) selon l'une quelconque des revendications précédentes, dans lequel le dispositif de sécurité (20) comprend en outre une unité de guidage configurée pour fournir une mise en prise coulissante entre la bague (30) et le capuchon de protection (50).

11. Dispositif de sécurité (20) selon l'une quelconque des revendications précédentes, dans lequel la bague (30) a une circonférence et dans lequel la liaison de pivot est prévue sur une partie de la circonférence.

12. Dispositif de sécurité (20) selon la revendication précédente et les revendications 2 à 10, dans lequel l'unité de verrouillage comprend deux évidements (34) prévus sur deux parties opposées de la circonférence de la bague (30).

13. Dispositif de sécurité (20) selon l'une quelconque des revendications précédentes, dans lequel le capuchon de protection (50) comprend un capuchon d'aiguille (60) adapté pour recevoir l'aiguille (14) et dans lequel le capuchon de protection (50) comprend une ouverture de capuchon (57) adaptée pour recevoir au moins une partie de la protection (40) dans la position fermée.

14. Raccord d'aiguille de sécurité adapté pour être fixé sur un dispositif médical, le raccord d'aiguille de sécurité comprenant une aiguille (14) et un dispositif de sécurité (20) selon l'une quelconque des revendications précédentes.

15. Dispositif médical adapté pour injecter et/ou retirer un fluide d'un corps, le dispositif médical comprenant une aiguille (14) et un dispositif de sécurité (20) selon l'une quelconque des revendications 1 à 13.

16. Raccord d'aiguille de sécurité selon la revendication 14 ou dispositif médical selon la revendication 15, dans lequel la protection (40) comprend un axe longitudinal parallèle à l'aiguille (14) dans la position fermée.
